# EUROPÄISCHE PATENTSCHRIFT

(11) **EP 0 557 833 B1**
(45) Veröffentlichungstag und Bekanntmachung des Hinweises auf die Patenterteilung: **21.08.1996**
(21) Anmeldenummer: 93102287.5
(22) Anmeldetag: 13.02.1993
(51) Int. Cl.: C07D 301/10

(54) **Verfahren zur Herstellung von Ethylenoxid**
Process for the preparation of ethylene oxide
Procédé de préparation d'oxyde d'éthylène

(30) Priorität: 20.02.1992 DE 4205090
(43) Veröffentlichungstag der Anmeldung: 01.09.1993
(73) Patentinhaber: BASF Aktiengesellschaft, 67063 Ludwigshafen (DE)
(72) Erfinder: Herzog, Klaus, Dr., W-6700 Ludwigshafen (DE); Boeck, Stefan, Dr., W-6700 Ludwigshafen (DE); Mross, Wolf Dieter, Dr., W-6710 Frankenthal (DE); Plueckhan, Juergen, Dr., W-6710 Frankenthal (DE); Boehning, Karl-Heinz, Dr., W-6700 Ludwigshafen (DE); Schwarzmann, Matthias, Dr., W-6703 Limburgerhof (DE)

(56) Entgegenhaltungen:
- EP-A- 0 428 845
- PATENT ABSTRACTS OF JAPAN vol. 5, no. 54 (C-50)(726) 15. April 1981

## Beschreibung

Die vorliegende Erfindung betrifft ein Verfahren zur Herstellung von Ethylenoxid durch die Oxidation von Ethylen mit Sauerstoff in Gegenwart silber- und promotorhaltiger Katalysatoren, wobei mindestens zwei Silberkatalysatoren unterschiedlicher Selektivität und Aktivität in einer kombinierten Katalysatorschüttung verwendet werden.

Ethylenoxid, eine wichtige Grundchemikalie, wird industriell durch die Oxidation von Ethylen mit Sauerstoff in Gegenwart silberhaltiger Katalysatoren hergestellt. Üblicherweise werden hierzu Trägerkatalysatoren verwendet, auf die das katalytisch aktive, metallische Silber mittels eines geeigneten Verfahrens aufgebracht worden ist (vgl. z.B. US-A 2 294 383, US-A 3 423 328, US-A 3 172 893). Als Trägermaterial wird im allgemeinen α-Aluminiumoxid verwendet, jedoch können prinzipiell auch andere poröse Materialien, wie Aktivkohle, Titan-, Zirkonium- oder Siliciumdioxide oder andere keramische Massen eingesetzt werden. Als Hilfsstoffe zur Verbesserung der katalytischen Eigenschaften werden insbesondere Alkali- oder Erdalkalimetallverbindungen, besonders bevorzugt Cäsiumverbindungen, in geringen Mengen auf den Träger aufgebracht (vgl. z.B. DE-A 23 00 512, DE-A 25 21 906, DE-A 27 53 359). Einige Schriften lehren die Verwendung anderer Zusatzstoffe, wie Wolfram und Molybdän (DE-A 24 54 972, JP-A 105 750 (1981), EP-A 357 293, EP-A 11 356). Ein anderer Dotierstoff zur Dotierung der Silberkatalysatoren zum Zwecke der Beeinflussung von deren Aktivität und Selektivität ist Rhenium (EP-A 266 015).

In JP-A 5471 (1981) wird ein Verfahren zur Herstellung von Ethylenoxid beschrieben, in dem alkalimetallhaltige Silberkatalysatoren verwendet werden, die entlang der Länge der Katalysatorschüttung mit unterschiedlichen Mengen an Alkalimetallen beladen sind, derart, daß entlang der Schüttung ein Alkalimetallkonzentrationsgradient vorliegt. Am oberen Ende der Katalysatorschüttung, d.h. an der Eintrittstelle des Reaktionsgases, soll nach dieser Schrift der Alkalimetallgehalt des Silberkatalysators niedriger sein als am unteren Ende der Katalysatorschüttung, also am Austritt des Reaktionsgases. Da die Alkalimetalldotierung des Silberkatalysators eine Abnahme der Aktivität und eine Erhöhung der Selektivität des Katalysators für die Bildung von Ethylenoxid zur Folge hat, kommt das Reaktionsgasgemisch beim Passieren des Reaktors mit zunehmend selektiverem und abnehmend aktiverem Silberkatalysator in Kontakt. Die mit diesem Verfahren erzielbaren Selektivitäten sind mit rund 70 % völlig unbefriedigend, weshalb dieses Verfahren bislang keine industrielle Bedeutung erlangt hat.

Katalysatoren, die Rhenium oder andere Übergangsmetallpromotoren enthalten, fanden bislang nicht die verbreitete industrielle Anwendung, wie sie aufgrund der guten Selektivitätswerte, die mit diesen neuartigen Katalysatoren erzielt werden, erwartet wurde. Ursächlich hierfür ist der Umstand, daß Zusatzstoffe zum katalytisch aktiven Silber des Katalysators dessen Aktivität senken, so daß zur Erzielung eines ausreichenden Umsatzes und somit zu einer wirtschaftlich zufriedenstellenden Ethylenoxidproduktion höhere Betriebstemperaturen im Reaktor eingestellt werden müssen, die in einem Bereich liegen, der in bestehenden technischen Anlagen aus konstruktiven Gründen nur schwierig zu erreichen ist.

Ein weiterer gravierender Nachteil der Anwendung hoher Betriebstemperaturen ist die durch sie bedingte Beschleunigung der Katalysatoralterung. Als Folge davon wird die Standzeit des Katalysators verringert, wodurch häufiger Katalysatorwechsel notwendig werden, was außer zu erhöhten Katalysatorkosten auch zu hohen Produktionsausfällen führt. Diese negativen Begleiterscheinungen sind bei Katalysatoren, die mit den, bezüglich der von ihnen bewirkten Selektivitätsverbesserung besonders empfehlenswerten Zusatzstoffen Molybdän, Wolfram und/oder Rhenium dotiert sind, besonders ausgeprägt.

Der vorliegenden Erfindung lag daher die Aufgabe zugrunde, ein Verfahren zur Herstellung von Ethylenoxid zu finden, das es ermöglicht, die Selektivitätsvorteile von Katalysatoren mit verringerter Aktivität zu nutzen ohne die Nachteile hoher Betriebstemperaturen in Kauf nehmen zu müssen und das auf diese Weise die Wirtschaftlichkeit der Ethylenoxidherstellung verbessert.

Dementsprechend wurde ein Verfahren zur Herstellung von Ethylenoxid durch die Oxidation von Ethylen mit Sauerstoff in Gegenwart silber- und promotorhaltiger Katalysatoren, wobei mindestens zwei Silberkatalysatoren unterschiedlicher Selektivität und Aktivität in einer kombinierten Katalysatorschüttung verwendet werden, gefunden, das dadurch gekennzeichnet ist, daß das Reaktionsgasgemisch beim Passieren des Reaktors mit zunehmend aktiveren und abnehmend selektiveren Silberkatalysatoren in Kontakt kommt.

Erfindungsgemäß werden also selektivere, aber inaktivere Silberkatalysatoren mit unselektiveren, aber aktiveren, kombiniert im Reaktor eingesetzt. Die Reihenfolge der Katalysatoren, wie sie vom Strom der Reaktionsgase durchströmt werden, geht dabei von den selektiveren zu den unselektiveren Katalysatortypen. Es können beliebig viele verschiedene Silberkatalysatoren miteinander in einer kombinierten Schüttung eingesetzt werden. Durch die Anordnung vieler Katalysatorschichten oder durch den Einsatz selektiverer und unselektiverer Silberkatalysatoren in bestimmten, sich über die Länge des Reaktionsrohres, d.h. über die Länge der Katalysatorschüttung ändernden Mischungsverhältnissen ist es auch möglich, einen Gradienten in der Katalysatorschüttung aufzubauen, so daß die Reaktionsgase beim Passieren der Katalysatorschüttung mit Silberkatalysatoren kontinuierlich abnehmender Selektivität in Kontakt kommen.

Die einfachste und am meisten bevorzugte Ausgestaltung des erfindungsgemäßen Verfahrens ist die Verwendung von nur zwei unterschiedlichen Silberkatalysatoren. Als unterschiedliche Silberkatalysatoren können solche Silberkatalysatoren, die sich hinsichtlich ihrer chemischen Zusammensetzung unterscheiden, verwendet werden, es ist aber auch vorteilhaft, Schüttungen von Silberkatalysatoren zu kombinieren, die sich lediglich in ihrer Aktivität und Selektivität bezüglich der Bildung von Ethylenoxid unterscheiden, beispielsweise in dem man eine Schüttung eines gebrauchten Silberkatalysators, der im Zuge seines Einsatzes zur Herstellung von Ethylenoxid an Selektivität und/oder Aktivität eingebüßt hat, mit einer Schüttung aus frisch produziertem Silberkatalysator ansonsten gleicher chemischer Zusammensetzung und auf die gleiche Weise hergestellt wie der gebrauchte Silberkatalysator, kombiniert.

Selbstverständlich können auch unterschiedliche Mischungen von Silberkatalysatoren erfindungsgemäß in kombinierten Katalysatorschüttungen zur Herstellung von Ethylenoxid verwendet werden. Die erfindungsgemäß kombinierten Schüttungen der Silberkatalysatoren können so erzeugt werden, daß man im einfachsten Fall einfach zwei oder mehr Lagen der unterschiedlichen Katalysatoren im Reaktionsrohr übereinander schichtet.

Es kann sich auch als besonders vorteilhaft auf die Selektivität der Ethylenoxidherstellung erweisen, wenn man die unterschiedlichen Katalysatoren in den kombinierten Katalysatorschüttungen im Reaktor während der Ethylenoxidherstellung auf unterschiedliche Temperaturen thermostatisiert, so daß jeder Silberkatalysator bei einer individuell einstellbaren Temperatur betrieben werden kann. Eine solche sogenannte strukturierte Temperaturführung kann auf einfache Weise mit Reaktoren bewerkstelligt werden, wie sie beispielsweise durch DE-A 22 01 528 und DE-A 28 30 765 zur Verfügung stehen.

Besonders einfach läßt sich mit den gängigen technischen Reaktoren zur Herstellung von Ethylenoxid diejenige Ausgestaltung des erfindungsgemäßen Verfahrens durchführen, bei der nur zwei unterschiedliche Katalysatoren in zwei Schüttungen miteinander kombiniert werden. Aufgrund der einfachen Ausführung und den sehr guten Ergebnissen, die sich auf diese Weise erzielen lassen, wird diese Ausführungsform des erfindungsgemäßen Verfahrens besonders bevorzugt. Bei dieser Verfahrensweise wird im allgemeinen eine solche Anordnung der unterschiedlichen Katalysatoren in der kombinierten Schüttung besonders bevorzugt, bei der der selektivere, aber inaktivere Katalysator zuerst mit den Reaktionsgasen in Berührung kommt, d.h. also näher zum Gaseingang der Reaktionsgase in den Reaktor liegt, wohingegen die Schüttung des aktiveren, aber unselektiveren Katalysators von den Reaktionsgasen vorteilhaft erst nach deren Durchtritt durch die Schicht des inaktiveren Katalysators passiert wird, also näher zum Gasausgang der Reaktionsgase aus dem Reaktor liegt.

Die erfindungsgemäßen kombinierten Schüttungen unterschiedlicher Silberkatalysatoren zur Herstellung von Ethylenoxid haben den Vorteil, daß die Selektivitätsgewinne, die mit dem Einsatz von selektiveren, aber inaktiveren Katalysatoren erreichbar sind, genutzt werden können, ohne daß der Nachteil der geringeren Aktivität gegenüber weniger selektiven, aber aktiveren Katalysatoren in Kauf genommen werden muß. Des weiteren können die bei alleiniger Verwendung von selektiveren, aber inaktiveren Silberkatalysatoren anzuwendenden hohen optimalen Betriebstemperaturen bei der Herstellung von Ethylenoxid bei Einsatz dieser Silberkatalysatoren in kombinierten Katalysatorschüttungen mit aktiveren, aber unselektiveren Silberkatalysatoren beträchtlich abgesenkt werden. Darüber hinaus hat die erfindungsgemäße Anwendung kombinierter Katalysatorschüttungen den ganz besonderen Vorteil, daß die Selektivität selektiver, aber inaktiver Silberkatalysatoren im Zuge der Betriebszeit wesentlich langsamer abfällt, als wenn ein solcher Katalysator allein, also nicht in kombinierter Schüttung mit einem anderen Katalysator eingesetzt wird, d.h. die Standzeit des betreffenden Katalysators wird beträchtlich verlängert. Diese Vorteile der Anwendung einer kombinierten Katalysatorschüttung führen zu einer beträchtlichen Verbesserung der Wirtschaftlichkeit des Verfahrens zur Ethylenoxid-Herstellung.

Als Silberkatalysatoren können im erfindungsgemäßen Verfahren alle zur Herstellung von Ethylenoxid aus Ethylen und Sauerstoff geeigneten silberhaltigen Trägerkatalysatoren verwendet werden. Als Trägermaterial kann prinzipiell jeder poröse Werkstoff dienen, der unter den Bedingungen der Ethylenoxidsynthese stabil ist, beispielsweise Aktivkohle, Aluminiumoxide, Titan-, Zirkonium- oder Siliciumdioxide oder andere keramische Massen.

Die geometrische Form der Trägerpartikel ist im allgemeinen von untergeordneter Bedeutung, zweckmäßigerweise sollten die Trägerpartikel aber Formen haben, die eine ungehinderte Diffusion der Reaktionsgase an einen möglichst großen Teil der mit den katalytisch aktiven, gegebenenfalls mit Zusatzstoffen dotierten Silberpartikeln belegten äußeren und inneren Oberfläche der Trägerpartikel ermöglicht.

Die katalytisch aktiven Bestandteile der Silberkatalysatoren wie sie im erfindungsgemäßen Verfahren angewandt werden, also Silber und gegebenenfalls zugesetzte Dotierstoffe, können mit allen Tränk- und Abscheideverfahren des Standes der Technik zur Herstellung von Silberkatalysatoren für die Herstellung von Ethylenoxid auf das Trägermaterial aufgebracht werden, wobei diese Verfahren eine oder mehrere Tränk- und Calcinationsstufen umfassen können. Beispielhaft seien die Herstellverfahren für Silberkatalysatoren genannt, wie sie in DE-A 23 00 512, DE-A 25 21 906, EP-A 14 457, EP-A 85 237, EP-A 384 312, DE-A 24 54 972, DE-A 33 21 895, EP-A 229 465, DE-A 31 50 205, EP-A 172 565 und EP-A 357 293 offenbart sind. Zur Dotierung der im erfindungsgemäßen Verfahren einsetzbaren Silberkatalysatoren mit Zusatzstoffen, die u.a. die Aktivität, Selektivität und Standzeit der Silberkatalysatoren beeinflussen, sogenannten Promotoren, gibt es prinzipiell keine Einschränkungen, d.h. es können sämtliche Promotoren des Standes der Technik zur Dotierung der Silberkatalysatoren verwendet werden. Als solche Promotoren seien insbesondere Alkalimetall- und Erdalkalimetallhydroxide oder -salze sowie die Verbindungen der Elemente der 6. und 7. Nebengruppe des Periodensystems, insbesondere Verbindungen der Elemente Wolfram, Molybdän und/oder Rhenium zu nennen.

Für die Anionen der Salze der Promotoren gibt es ebenfalls keine Beschränkungen, beispielsweise können alle Halogenide, insbesondere Fluorid, Chlorid, Carboxylate, Nitrat, schwefelhaltige Anionen, wie Sulfat oder Sulfid, Phosphate, Cyanid, Hydroxid, Carbonate oder Anionen von Heteropolysäuren, insbesondere von Heteropolysäuren der Elemente der 6. und 7. Nebengruppe des Periodensystems, besonders bevorzugt Anionen von Heteropolysäuren des Wolframs, Molybdäns und/oder Rheniums, Anionen dieser Salze sein.

Beispielhaft für die mit Promotoren dotierten Silberkatalysatoren, die im erfindungsgemäßen Verfahren eingesetzt werden können, seien die Silberkatalysatoren von DE-A 23 00 512, DE-A 25 21 906, EP-A 14 457, DE-A 24 54 972, EP-A 172 565, EP-A 357 293, EP-A 266 015, EP-A 11 356, EP A 85 237, DE-A 25 60 684 und DE-A 27 53 359 genannt. Zur besseren Veranschaulichung, was für Katalysatoren im erfindungsgemäßen Verfahren eingesetzt werden können, seien beispielhaft Silberkatalysatoren mit einem Silbergehalt von 5 bis 50 Gew.-%, insbesondere von 6 bis 30 Gew.%, bezogen auf den Gesamtkatalysator, einem Gehalt an den schweren Alkalimetallen Rubidium und/oder Cäsium von 1 bis 5000 Gew.-ppm, einem Gehalt an Wolfram von 1 bis 5000 Gew.-ppm, einem Gehalt an Molybdän von 1 bis 3000 Gew.-ppm und/oder einem Gehalt an Rhenium von 1 bis 10 000 Gew.-ppm, bezogen auf den Gesamtkatalysator genannt. Das zur Herstellung solcher Silberkatalysatoren vorteilhaft verwendete Trägermaterial α-Aluminiumoxid hat vorzugsweise eine BET-Oberfläche, bestimmt nach Brunauer et al, J. Am. Chem. Soc. 60, S. 309 (1938), von 0,1 bis 20 m²/g, eine Porosität gemessen nach dem Verfahren der Quecksilberporosimetrie von 10 % bis 90 % und eine Kaltwasseraufnahme bei 20°C innerhalb von 5 Minuten von 0,1 bis 0,9 ml/g.

Die in den im vorangegangenen Absatz beispielhaft genannten Schriften offenbarten Silberkatalysatoren mit den darin angegebenen Gehalten an Silber und Promotoren, hergestellt nach den darin angegebenen Imprägnier-, Trocknungs-, Silberzersetzungs- und Calcinierverfahren, können allesamt mit gutem Erfolg im erfindungsgemäßen Verfahren eingesetzt werden. Für den Erfolg des erfindungsgemäßen Verfahrens ist somit nicht die Art der jeweils eingesetzten Katalysatoren kritisch, sondern allein die Maßnahme, daß kombinierte Schüttungen von Katalysatoren, die sich bezüglich ihrer Selektivität und Aktivität unterscheiden, verwendet werden. Allein durch diese Maßnahme können die vorher erwähnten Vorteile, z.B. die Verlängerung der Standzeit der betreffenden Silberkatalysatoren, erzielt werden.

Mit Hilfe der erfindungsgemäßen kombinierten Katalysatorschüttungen der Silberkatalysatoren kann Ethylenoxid nach an sich herkömmlichen Methoden durch die Direktoxidation von Ethylen mit Sauerstoff erzeugt werden. Dazu können alle in den Ethylenoxidherstellungsverfahren des Standes der Technik anwendbaren Reaktoren verwendet werden, beispielsweise die üblicherweise industriell eingesetzten außengekühlten Rohrbündelreaktoren (vgl. Ullmann's Encyclopedia of Industrial Chemistry; 5th Ed.; Vol. A10; S. 117-135, 123-125; VCH Verlagsgesellschaft; Weinheim 1987) als auch Reaktoren mit loser Katalysatorschüttung und Kühlrohren, beispielsweise die Reaktoren gemäß DE-A 34 14 717, EP-A 82 609 und EP-A 339 748. Zur Erzeugung der kombinierten Katalysatorschüttung werden die unterschiedlichen Katalysatoren einfach nacheinander, in der gewünschten Reihenfolge, in den betreffenden Reaktor eingefüllt.

Zur Herstellung von Ethylenoxid aus Ethylen und Sauerstoff mit Hilfe der erfindungsgemäßen Katalysatorschüttungen kann unter herkömmlichen Reaktionsbedingungen, wie sie beispielsweise in DE-A 25 21 906, EP-A 14 457, DE-A 23 00 512, EP-A 172 565, DE-A 24 54 972, EP-A 357 293, EP-A 266 015, EP-A 85 237, EP-A 82 609 und EP-A 339 748 beschrieben sind, gearbeitet werden. Dem Ethylen und molekularen Sauerstoff enthaltenden Reaktionsgas können dabei zusätzlich noch Inertgase wie Stickstoff oder sich unter den Reaktionsbedingungen inert verhaltende Gase, wie Wasserdampf, Methan sowie gewünschtenfalls Reaktionsmoderatoren (Inhibitoren), beispielsweise halogenierte Kohlenwasserstoffe, wie Vinylchlorid oder 1,2-Dichlorethan, zugemischt werden. Zweckmäßigerweise liegt der Sauerstoffgehalt des Reaktionsgases in einem Bereich, in dem keine explosionsfähigen Gasgemische vorliegen. Eine geeignete Zusammensetzung des Reaktionsgases zur Herstellung von Ethylenoxid kann z.B. aus ca. 30 Vol.-% Ethylen, ca. 8 Vol.-% Sauerstoff, 0,5 bis 5 ppm eines chlorhaltigen Inhibitors, wie Vinylchlorid oder Dichlorethan, bestehen, wobei der Rest des Reaktionsgases in der Regel aus Kohlenwasserstoffen, wie Methan oder Ethan, oder aber auch aus Inertgasen, wie Stickstoff, zusammengesetzt sein kann. Zusätzlich können auch noch andere Stoffe wie Wasserdampf, Kohlendioxid oder Edelgase im Reaktionsgas enthalten sein. Die Oxidation wird im allgemeinen bei Temperaturen von 165 bis 300°C durchgeführt.

Vorteilhafterweise kann die Herstellung von Ethylenoxid aus Ethylen und Sauerstoff in einen Kreisprozeß durchgeführt werden. Dabei wird das Reaktionsgasgemisch im Kreislauf durch den Reaktor geleitet und nach jedem Durchgang das neugebildete Ethylenoxid sowie die bei der Umsetzung gebildeten Nebenprodukte aus dem Produktgasstrom entfernt, der nach Ergänzung mit den erforderlichen Mengen an Ethylen, Sauerstoff und Reaktionsmoderatoren wieder in den Reaktor zurückgeführt wird. Die Abtrennung des Ethylenoxids aus dem Produktgasstrom und seine Aufarbeitung können nach den üblichen Verfahren des Standes der Technik (vgl. Ullmann's Encyclopedia of Industrial Chemistry; 5th Ed.; Vol. A10; S. 117-135, 123-125; VCH Verlagsgesellschaft; Weinheim 1987) erfolgen.

### Beispiele

Die vergleichenden Versuche zur Bestimmung der Selektivität und Aktivität der einzelnen Silberkatalysatoren und der erfindungsgemäßen kombinierten Katalysatorschüttungen wurden folgendermaßen durchgeführt:

Die Katalysatoren wurden unzerkleinert in einer Gesamtmenge von 13 dm³ in einen stählernen Reaktor gefüllt. Der Reaktor wurde von einem Kühlmantel umhüllt, durch den die Thermostatisierflüssigkeit geleitet wurde. Als Reaktionsgas diente ein Gas der folgenden Zusammensetzung:
30 Vol.-% Ethylen
8 Vol.-% Sauerstoff
ca. 6,5 Vol.-% Kohlendioxid
ca. 4 Vol.-% Argon
ca. 4 Vol.-% Wasserdampf
ca. 3 ppm Vinylchlorid
Rest Methan

Die Bezeichnung "ca." bedeutet, daß der Gehalt dieser Komponenten im Reaktionsgasstrom während der Dauer der Testperiode um ± 20 % schwanken kann.

Der Druck im Reaktor wurde auf 16 bar eingestellt. Die Temperatur in der Kühlflüssigkeit wurde so eingestellt, daß bei einer Raumgeschwindigkeit von 3300 Nm³/m³ Katalysator x h ein Sauerstoffumsatz von 35 % erzielt wurde. Nach 4 und nach 50 Tagen Dauerbetrieb wurden Proben gezogen und die Selektivität der Oxidation des Ethylens zu Ethylenoxid und die Aktivität des Katalysators, ausgedrückt als die Temperatur, welche zur Erzielung eines 35 %igen Sauerstoffumsatzes notwendig ist, bestimmt.

### Katalysatoren:

Als Trägermaterial für alle eingesetzten Katalysatoren diente α-Aluminiumoxid einer Reinheit von mehr als 99 %. Der Träger wurde in Form von Ringen mit den Abmessungen 8 x 8 x 2 mm (Gesamtdurchmesser x Höhe x Wandstärke) eingesetzt. Die Kenndaten des verwendeten Trägermaterials sind in Tabelle I aufgelistet.

**Tabelle I**

| Kenndaten des Trägermaterials | |
|---|---|
| Gehalt an Si (Gew.-%) | 0.28 |

| lösliche Ionen (Gew.-%) | |
|---|---|
| Al | 370 |
| Ca | 170 |
| K | 80 |
| Na | 84 |
| BET-Oberfläche (m²/g) | 0.8 |
| Kaltwasseraufnahme (20°C, ml/g) | 0.42 |
| Porosität (%) | 56 |
| mittlerer Porendurchmesser (µm) | 13.0 |
| Abrieb (Gew.-%) | 2.7 |

Die Bestimmung des Gehaltes an löslichen Ionen wurde so durchgeführt, daß man ca. 10 g Trägerpartikel genau abwog und anschließend diese mit halbkonzentrierter Salpetersäure 10 Minuten lang kochte. Die filtrierte Lösung des so erhaltenen Extraktes wurde zur quantitativen Bestimmung der genannten Elemente mittels Atomabsorptionsspektrometrie verwendet. Der SiO₂-Gehalt des α-Aluminiumoxids wurde photometrisch durch die Messung der Extinktion des blauen Silicium-Molybdato-Komplexes, der sich nach alkalischem Aufschluß eine Probe des Trägermaterials und Umsetzung der erhaltenen Lösung mit Ammoniumheptamolybdat bildet, bei der Wellenlänge von 820 nm bestimmt.

### Katalysator A:

100 Gew.-Teile des genannten Trägermaterials wurden bei Raumtemperatur mit einer Lösung, enthaltend 27,4 Gew.-Teile Silbernitrat, 0,292 Gew.-Teile Lithiumnitrat, 0,0729 Gew.-Teile Cäsiumhydroxid, 24,2 Gew.-Teile sek.-Butylamin und 5,9 Gew.-Teile Wasser, getränkt. Anschließend wurde der so imprägnierte Träger in einem Bandcalcinierofen bei 220°C binnen 10 Minuten zum fertigen Katalysator umgewandelt.

### Katalysator B:

Nach der für die Herstellung von Katalysator A beschriebenen Arbeitsweise wurde auch Katalysator B unter Verwendung der folgenden Einsatzstoffe hergestellt:

Auf 100 Gew.-Teile Träger wurde eine Tränklösung bestehend aus 27,5 Gew.-Teilen Silbernitrat, 0,292 Gew.-Teilen Lithiumnitrat, 0,0729 Gew.-Teilen Cäsiumhydroxid, 0,0106 Gew.-Teilen Molybdäntrioxid, dieses gelöst in 1 Gew.-Teil einer 10 gew.%igen wäßrigen Ammoniaklösung, 24,2 Gew.-Teilen sek.-Butylamin und 4,9 Gew.-Teilen Wasser aufgebracht.

In Tabelle II sind die Gehalte der Katalysatoren A und B an katalytisch aktiven Metallen aufgelistet:

**Tabelle II**

| Gehalte an katalytisch aktiven Metallen der Katalysatoren A und B | | |
|---|---|---|
| Katalysator | A | B |
| Ag [Gew.-%] | 14.8 | 14.8 |
| Cs [ppm] | 550 | 550 |
| Li [ppm] | 250 | 250 |
| Mo [ppm] | 0 | 60 |

Die Katalysatoren A und B wurden wie beschrieben einzeln auf ihre Selektivität und Aktivität bei der Herstellung von Ethylenoxid aus Ethylen und Sauerstoff untersucht. Die Ergebnisse dieser Versuche sind in Tabelle III unter den Rubriken A und B aufgelistet.

### Beispiel 1 (erfindungsgemäß)

Im Reaktionsrohr wurde eine Schicht aus Katalysator A und eine Schicht gleicher Menge an Katalysator B aufgeschüttet, wobei Katalysator A die zum Gasausgang hin gelegene Hälfte der Katalysatorschüttung einnahm. Die mit dieser kombinierten Schüttung erzielten Ergebnisse bei der Ethylenoxid-Herstellung sind in Tabelle III unter der Rubrik 1 aufgelistet.

### Beispiel 2 (erfindungsgemäß)

Die in diesem Versuch eingesetzte kombinierte Katalysatorschüttung bestand zu einem Drittel aus Katalysator A und zu zwei Dritteln aus Katalysator B, wobei die Schicht des Katalysators A, das zum Gasausgang hin gelegene Drittel der kombinierten Katalysatorschüttung einnahm. Die mit dieser kombinierten Schüttung erzielten Versuchsergebnisse sind in Tabelle III unter der Rubrik 2 aufgelistet.

**Tabelle III**

| Testergebnisse | | | | |
|---|---|---|---|---|
| Katalysator | A | B | 1 | 2 |
| | | | | |

| nach vier Tagen Betriebsdauer: | | | | |
|---|---|---|---|---|
| S [%] | 80.2 | 81.4 | 81.0 | 81.2 |
| a [°C] | 219 | 237 | 222 | 228 |

| nach fünfzig Tagen Betriebsdauer: | | | | |
|---|---|---|---|---|
| S [%] | 80.0 | 80.8 | 80.8 | 80.9 |
| a [°C] | 226 | 245 | 229 | 235 |

| Verluste an Aktivität und Selektivität: | | | | |
|---|---|---|---|---|
| S [%] | - 0.2 | - 0.6 | - 0.2 | - 0.3 |
| a [°C] | - 7 | - 8 | - 7 | - 7 |
| S: Selektivität a: Aktivität | | | | |

### Vergleichsbeispiel:

Um zu zeigen, daß die vorteilhaften Effekte der kombinierten Katalysatorschüttung nicht auf die durch die verkürzte Schüttung von Katalysator A verkürzte Verweilzeit bei gleichzeitig erhöhter Reaktionstemperatur zurückzuführen sind, wurde der im folgenden Absatz beschriebene Vergleichsversuch durchgeführt. Die in diesem Vergleichsversuch angewandte Temperatur lag deutlich unterhalb der optimalen Betriebstemperatur von Katalysator B (237°C), so daß dieser Katalysator, wäre er anstelle von Katalysator A in diesem Vergleichsversuch eingesetzt worden, nur in völlig unzureichendem Maße, d.h. praktisch nicht wirksam gewesen wäre.

Im Reaktionsrohr wurde eine Schicht aus einer unter den Reaktionsbedingungen inerten Schüttung aus α-Aluminiumoxid und eine Schüttung bestehend aus Katalysator A aufgeschüttet. Die α-Aluminiumoxid-Schüttung nahm dabei das zum Gaseingang hin gelegene Drittel der Schüttung ein, wohingegen Katalysator A die zum Gasausgang hin gelegenen zwei Drittel der Schüttung in Anspruch nahm.

Zur Herstellung von Ethylenoxid unter den beschriebenen Bedingungen wurde zur Erzielung eines 35 %igen Sauerstoffumsatzes nach 4 Tagen Betriebsdauer eine Temperatur von 228°C benötigt (≙ der Aktivität a), wobei eine Selektivität für Ethylenoxid von 80,4 % erzielt wurde.

### Diskussion der Ergebnisse der Beispiele:

Aus Tabelle III sind die Vorteile der Anmeldung einer kombinierten Schüttung verglichen mit dem Einsatz einer einzelnen Katalysatorspecies klar ersichtlich:

Der Vorteil der kombinierten Katalysatorschüttungen gemäß Beispiel 1 und 2, welche zum einen Teil aus einem Katalysator bestehen, der selektivitätssteigernde und aktivitätssenkende Promotoren enthält, zum anderen Teil aus einem lediglich mit Alkalimetallen dotierten Silberkatalysator, gegenüber einer Schüttung aus dem molybdändotierten Katalysator B liegt in der deutlich besseren Aktivität und in dem über die Betriebsdauer gesehen, geringeren Verlust an Selektivität. Dadurch kann, wie sich aus dem Vergleich von Katalysator B mit der kombinierten Katalysatorschüttung von Beispiel 2 ergibt, die Selektivität des allein eingesetzten selektiveren, aber inaktiveren Katalysators nach einer gewissen Betriebsdauer (50 Tage) auch durch die kombinierte Schüttung unterschiedlich selektiver und aktiver Katalysatoren erzielt werden und dies bei einer deutlich höheren Aktivität.

## Patentansprüche

1. Verfahren zur Herstellung von Ethylenoxid durch die Oxidation von Ethylen mit Sauerstoff in Gegenwart silber- und promotorhaltiger Katalysatoren, wobei mindestens zwei Silberkatalysatoren unterschiedlicher Selektivität und Aktivität in einer kombinierten Katalysatorschüttung verwendet werden, dadurch gekennzeichnet, daß das Reaktionsgemisch beim Passieren des Reaktors mit zunehmend aktiveren und abnehmend selektiveren Silberkatalysatoren in Kontakt kommt.

2. Verfahren nach Anspruch 1, dadurch gekennzeichnet, daß man mindestens zwei mit Promotoren unterschiedlich dotierte Silberkatalysatoren verwendet.

3. Verfahren nach Anspruch 1, dadurch gekennzeichnet, daß einer oder mehrere der verwendeten Silberkatalysatoren mit mindestens einem Element der 6. oder 7. Nebengruppe des Periodensystems dotiert ist.

4. Verfahren nach Anspruch 1, dadurch gekennzeichnet, daß mindestens einer der Silberkatalysatoren mit Wolfram, Molybdän und/oder Rhenium dotiert ist.

5. Verfahren nach Anspruch 1, dadurch gekennzeichnet, daß mindestens einer oder mehrere der verwendeten Silberkatalysatoren mit mindestens einem Alkalimetall dotiert ist.

6. Verfahren nach Anspruch 1, dadurch gekennzeichnet, daß man die verschiedenen Silberkatalysatoren in der kombinierten Katalysatorschüttung im Reaktor in mindestens zwei Schichten anordnet.

7. Verfahren nach Anspruch 1, dadurch gekennzeichnet, daß man die einzelnen Silberkatalysatoren in den einzelnen Schichten der kombinierten Katalysatorschüttung auf unterschiedliche Temperaturen thermostatisiert.

## Claims

1. A process for preparing ethylene oxide by oxidizing ethylene with oxygen in the presence of silver- and promoter-containing catalysts using at least two silver catalysts of different selectivity and activity in a combined catalyst bed, wherein the reaction mixture comes into contact with silver catalysts of increasing activity and decreasing selectivity on passage through the reactor.

2. A process as claimed in claim 1, wherein at least two silver catalysts doped with different promoters are used.

3. A process as claimed in claim 1, wherein one or more of the silver catalysts is doped with at least one element of group VIb or VIIb of the periodic table.

4. A process as claimed in claim 1, wherein at least one of the silver catalysts is doped with tungsten, molybdenum and/or rhenium.

5. A process as claimed in claim 1, wherein at least one of the silver catalysts is doped with at least one alkali metal.

6. A process as claimed in claim 1, wherein the various silver catalysts are arranged in at least two layers in the combined catalyst bed in the reactor.

7. A process as claimed in claim 1, wherein the individual silver catalysts in the individual layers in the combined catalyst bed are maintained at different temperatures.

## Revendications

1. Procédé de préparation de l'oxyde d'éthylène par l'oxydation de l'éthylène avec de l'oxygène en présence de catalyseurs contenant de l'argent et des promoteurs, où on utilise au moins deux catalyseurs à l'argent d'activité et de sélectivité différentes dans une garniture ou masse de catalyseur combinée caractérisé en ce que le mélange de réaction entre en contact au cours de son passage à travers le réacteur avec des catalyseurs à l'argent à activité croissante et à sélectivité décroissante.

2. Procédé suivant la revendication 1, caractérisé en ce que l'on utilise au moins deux catalyseurs à l'argent dopés différemment de promoteurs.

3. Procédé suivant la revendication 1, caractérisé en ce que le seul ou les plusieurs catalyseurs à l'argent utilisés sont dopés d'au moins un élément des sixième ou septième sous-groupe du système périodique.

4. Procédé suivant la revendication 1, caractérisé en ce qu'au moins l'un des catalyseurs à l'argent est dopé de tungstène, de molybdène et/ou de rhénium.

5. Procédé suivant la revendication 1, caractérisé en ce que le seul ou les plusieurs catalyseurs à l'argent utilisés sont dopés avec au moins un métal alcalin.

6. Procédé suivant la revendication 1, caractérisé en ce que l'on agence les divers catalyseurs à l'argent dans la masse ou garniture de catalyseurs combinée en au moins deux couches dans le réacteur.

7. Procédé suivant la revendication 1, caractérisé en ce que l'on procède à la thermostatisation des couches individuelles de la masse ou de la garniture de catalyseur combinée à des températures différentes.
